# EUROPÄISCHE PATENTSCHRIFT

(11) **EP 0 930 865 B1**
(45) Veröffentlichungstag und Bekanntmachung des Hinweises auf die Patenterteilung: **12.06.2002**
(21) Anmeldenummer: 97941859.7
(22) Anmeldetag: 30.08.1997
(51) Int. Cl.: A61K 6/083

(54) **ZUSAMMENSETZUNG UND METHODE ZUR ZAHNRESTAURIERUNG**
DENTAL RESTORATION COMPOSITION AND METHOD
COMPOSITION ET PROCEDE DE RESTAURATION DENTAIRE

(30) Priorität: 18.09.1996 DE 19638068
(43) Veröffentlichungstag der Anmeldung: 28.07.1999
(73) Patentinhaber: Mühlbauer, Wolfgang Carl Friedrich, Dr., 22609 Hamburg (DE)
(72) Erfinder: Mühlbauer, Wolfgang Carl Friedrich, Dr., 22609 Hamburg (DE)
(74) Vertreter: Schupfner, Gerhard D., Dr. Dipl.-Chem.
(86) Internationale Anmeldenummer: DE9701906
(87) Internationale Veröffentlichungsnummer: WO9811862

(56) Entgegenhaltungen:
- EP-A- 0 219 058
- EP-A- 0 220 551
- EP-A- 0 554 890
- WO-A-88/05651
- WO-A-93/12759
- WO-A-94/13221
- DE-A- 3 903 703

## Beschreibung

Die Erfindung betrifft lichthärtbare Zusammensetzungen, sogenannte polymerisierbare Zemente, im Folgenden Compomere genannt und ein Verfahren zu deren direkter Applikation in eine Zahnkavität.

Nachdem Amalgame aufgrund der Quecksilberbelastung immer weniger eingesetzt werden, nimmt die Bedeutung von Kunststofffüllungen zur Restaurierung eines Zahns zu. Hierzu werden insbesondere hochviskose Pasten eingesetzt. Materialien, wie sie unter anderem in der EP 0 486 775 beschrieben werden, bestehen aus einer Harzmatrix und einer Füllstoffmischung, die eine sehr feste Konsistenz ergeben.

Andere Mischungen enthalten neben der üblichen Harzmischung zusätzlich polymerisierbare Säuren und als Füllstoffzusatz Ionen freisetzende Füllstoffe, z.B. Glasionomer-Zementmischungen, wie in der EP 0 219 058 beschrieben. Ausführungsbeispiele betreffend die polymerisierbaren Säuren sind unter anderem in der EP 0 554 890, der EP 0 425 200 und der EP 0 234 934 beschrieben.

Die Pasten, die sich nach oben beschriebenen Verfahren ergeben, sind standardmäßig sehr fest, ja zum Teil sogar stopfbar. Daraus ergeben sich Applikationsinstrumente, wie z.B. in der EP 0 657 143 beschrieben, die aus einer Kolbenspritze bestehen, die eine verengungsfreie Ausbringöffnung aufweist.

Es sind auch dünnflüssige Systeme bekannt, die sich aber nur als Befestigungszemente eignen, wie z.B. in der EP 0 486 774 beschrieben.

In der US 5,547,379 wird eine Composite-Mischung beschrieben. Sie enthält 30 bis 70% Aerosile bezogen auf die Füllstoffmenge, mindestens insgesamt 30% Füllstoff und eine in Composites üblicherweise verwendete Harzmischung. Eine Paste mit einem so großen Aerosil-Anteil ist schwer herzustellen und hat aufgrund des hohen Aerosil-Anteils einen geringen Gesamtfüllstoffanteil und damit einen hohen Schrumpf.

Es besteht daher die Aufgabe, eine Mischung in der Konsistenz einer fließfähigen Paste zur Restaurierung eines Zahns herzustellen, die durch eine dünne Kanüle direkt in die Kavität appliziert werden kann und die trotz fließfähiger Konsistenz möglichst schrumpfarm sein soll.

Die vorliegende Erfindung ist durch den Hauptanspruch definiert. Die Unteransprüche beziehen sich auf bevorzugte Ausführungsformen.

Die Erfindung stellt eine Zusammensetzung bereit aus einer dünnfließenden Paste und ein Verfahren, diese durch eine Kanüle direkt in die Kavität zu applizieren. Diese Mischung besteht aus einer lichthärtbaren Harzmatrix, die polymerisierbare Säure enthält und einer Füllstoffmischung, die unter anderem einen Ionen freisetzenden Füllstoff enthält.

Überraschenderweise ist ein solches System auch zu realisieren, wenn die Füllstoffmischung nur einen wesentlich geringeren Anteil an mikrofeinem Siliciumdioxid enthält als in US 5,547,379 beschrieben. Damit kann der Gesamtfüllstoffgehalt heraufgesetzt werden und das Produkt ist leichter herzustellen. Hinzu kommt, daß die Compomer-Zusammensetzung mit ihrem relativ zu einem Composite veränderten Eigenschaftsprofil für diesen Zweck besonders geeignet ist. Hier kommt z. B. die Eigenhaftung an der Zahnsubstanz in Zusammenhang mit der erhöhten Fließfähigkeit besonders gut zum Tragen. Besonders vorteilhaft ist es auch, daß durch Expansion der Schrumpf praktisch aufgefangen wird.

Auch die Fluoridfreisetzung, die das Material bakteriostatisch macht, bringt diesem Material gegenüber einem normalen fließfähigen Composite Vorteile.

Die Fließfähigkeit der Mischung und das entsprechende Applikationsinstrument ermöglichen es, auch die kleinsten Risse und Höhlen innerhalb der Kavität 100%ig auszufüllen. Da diese Mischungen sehr fließfähig sind, ist eine Aushärtung in mehreren Schichten ohne Probleme möglich. Dadurch und insbesondere durch den Einsatz von Verbindungen aus der Klasse der polymerisierbaren Zementmischungen ist es möglich, eine Restauration fast ohne Randspalt und ohne innere Hohlräume zu gewährleisten.

Auch eine Applikation durch eine enge Öffnung hindurch ist mit dieser Technik möglich.

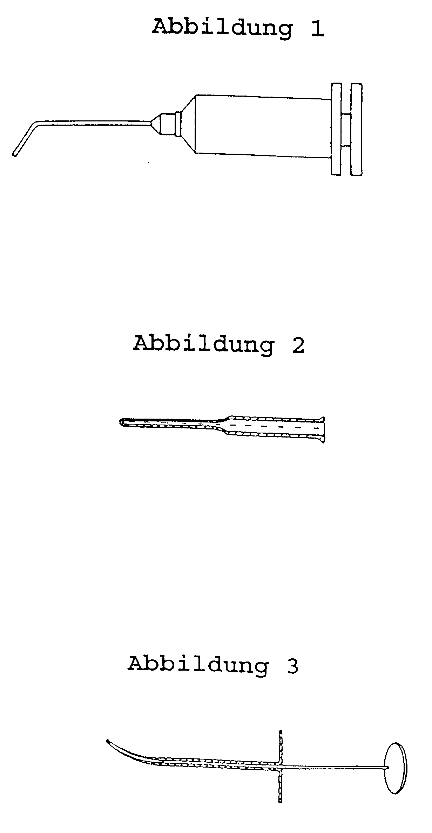

Als Applikationsinstrument bieten sich neben einer Spritze mit aufgesetzter auswechselbarer Kanüle, siehe Abbildung 1, auch solche Container an, die in sich schon eine feine Kanüle aufweisen, siehe Abbildung 2; ferner auch wie z.B. in DE 8 528 512 beschrieben, siehe Abbildung 3, und auch die in US 5,336,088 aufgezeigte Applikationsform.

Der Durchmesser der Kanüle bzw. der Austrittsöffnung ist kleiner 1,3 mm und liegt bevorzugt zwischen 0,2 und 0,6 mm, besonders bevorzugt 0,4 bis 0,55 mm.

Für die Harzmischung eines derartigen fließfähigen Compomers sind alle gängigen (Meth)-Acrylate geeignet, insbesondere Methacrylate bzw. Dimethacrylate wie Bis-[4-(2-hydroxy-3-methacryloyloxy propoxy)-phenyl]-dimethylmethan (BisGMA), Ethylenglycoldimethacrylat, Diethylenglycoldimethacrylat, Triethylenglycoldimethacrylat, Tetraethylenglycoldimethacrylat, Diurethandimethacrylat, Hydroxyethylmethacrylat (HEMA), Hydroxypropylmethacrylat, Hydroxypropandimethacrylat, Dihydroxypropanmethacrylat (GMA), ethoxyliertes Bisphenol A dimethacrylat, Bisphenol A dimethacrylat, Butandioldimethacrylat, Hexandioldimethacrylat, Dekandioldimethacrylat, Dodekandioldimethacrylat, Trimethylolpropantrimethacrylat, Trimethylpropantrimethacrylat, Tetramethacrylamido-n-pentan.

Als polymerisierbare Säuren eignen sich methacrylierte Polyacrylsäuren, methacrylierte Oligo- bzw. Polymaleinsäuren, wie aus EP 0 218 248 bekannt, methacrylierte Phosphate, Phosphonate, Diphosphonate und Diphosphate insbesondere mit Hydroxyethylmethacrylat oder mit Dihydroxypropanmethacrylat veresterte Phosphonate und Phosphate sowie entsprechende Sulfate.

Auch partiell mit HEMA oder GMA veresterte Fumarsäure, Maleinsäure, Itakonsäure, Bernsteinsäure, Weinsäure, Citronensäure, Phthalsäure, Terephthalsäure, Pyromillitsäure oder Benzoltricarbonsäure sind geeignet.

Es können statt des zitierten Ethylenglycol- (= HEMA) oder Glycerinspacers (= GMA), der die Verbindung zwischen der Säuregruppe und der Methacrylategruppe darstellt, auch längerkettige Spacer verwendet werden.

Als Starter sind besonders α-Diketone in Zusammenhang mit Aminen geeignet.

Die Füllstoffmischung enthält 1 bis 5% eines mikrofeinen Siliciumdioxids. Geeignet sind pyrogene Kieselsäuren mit einer Oberfläche von 50 bis 300 m²/g. Die Primärteilchengröße beträgt 40 bis 150 nm. Besonders bevorzugt sind pyrogene Kieselsäuren mit einer Oberfläche von 50 bis 300 m²/g mit hydrophobierter Oberfläche. Es sind aber auch Fällungskieselsäuren geeignet.

Der gröbere Teil der Füllstoffmischung enthält einen Ionen freisetzenden Füllstoff, bevorzugt einen Fluorid und Kationen freisetzenden Füllstoff.

Besonders bevorzugt sind die Pulver von Ionomerzementen, wie sie klassisch in GB 1 504 520 oder GB 1 316 129 beschrieben sind, wobei sich fluoridangereicherte Formen mit bis zu 20% Fluorid besonders gut eignen.

Auch lonomerzemente mit röntgenopaken Elementen, z.B. Barium, Lanthan, Zirkon, Zink oder das in EP 0 244 959 beschriebene Strontium, sind einsetzbar. Die Korngröße eines derartigen Ionomerzementes sollte zwischen 0,5 bis 10,0 µm mittlerer Korngröße liegen, bevorzugt zwischen 0,7 und 6,0 µm mittlerer Korngröße.

Der Füllstoff kann in silanisierter ggf. auch unsilanisierter Form vorliegen.

Als inerte, röntgenopake Füllstoffe können inerte d.h. hydrolysestabile Gläser eingesetzt werden. Besonders geeignet sind Bariumaluminumsilikatgläser oder auch Strontiumaluminumsilikatgläser der Korngröße 0,5 bis 8 µm mittlerer Korngröße, bevorzugt 0,7 bis 4,0 µm, die bevorzugt in silanisierter Form eingearbeitet werden sollten.

Auch andere röntgenopake Füllstoffe, wie Oxide und Fluoride von Seltenen Erden sind einsetzbar.

Beispiele geeigneter fließfähiger Mischungen gemäß der Erfindung sind

| **Mischung I** | |
|---|---|
| Bernsteinsäuremonomethacrylester | 15% |
| Hexandioldimethacrylat | 18% |
| Hydroxyethylmethacrylat | 4,7% |
| Ionomer Glas von der Firma ITC* | 50% |
| Barium Glas von der Firma Schott # | 10% |
| Aerosil R972 von der Firma Degussa | 2% |
| Campherchinon | 0,1% |
| Amin | 0,3% |

| | |
|---|---|
| * mittlere Korngröße 4 µm | |
| # mittlere Korngröße 1 µm | |

| **Mischung II** | |
|---|---|
| Butandioldimethacrylat | 15 % |
| Bis-(2-Methacryloxyethyl)phosphat | 9% |
| Triethylenglycoldimethycrylat | 8% |
| Hydroxyethylmethacrylat | 7% |
| Barium-Ionomer Glas von der Firma ITC* | 50% |
| GlasIonomer Type II der Firma Shofu | 10% |
| Aerosil R972 von der Firma Degussa | 1,6% |
| Campherchinon | 0,1% |
| Amin | 0,3% |

| | |
|---|---|
| * mittlere Korngröße 1 µm | |

| **Mischung III** | |
|---|---|
| Bernsteinsäuremonomethacrylester | 7,6 % |
| Bis-(2-Methacryloxyethyl)phosphat | 7,6 % |
| Hexandioldimethacrylat | 11,4 % |
| Urethandioldimethacrylat | 11,4 % |
| Ionomer Glas von der Firma ITC* | 60% |
| Aerosil R972 von der Firma Degussa | 1,6 % |
| Campherchinon | 0,1 % |
| Amin | 0,3 % |

| | |
|---|---|
| * mittlere Korngröße 4 µm | |

Zur Applikation wird die dünnfließende Mischung I, II oder III direkt in die Kavität eingegeben. Sie verteilt sich dort selbständig durch leichtes Bewegen als dünne Schicht. Diese kann dann mit sichtbarem Licht ausgehärtet werden. Eine zweite Schicht der Compomermischung wird hinzugefügt, und es erfolgt abermals eine Lichthärtung. Dieses Verfahren wird wiederholt, bis die gesamte Kavität ausgefüllt ist. Vorteilhafter ist, die Kavität vorher mit einem Haftvermittler z.B. SOLIST® der Firma DMG, Hamburg einzupinseln. Danach erfolgt eine Schichtung wie oben beschrieben.

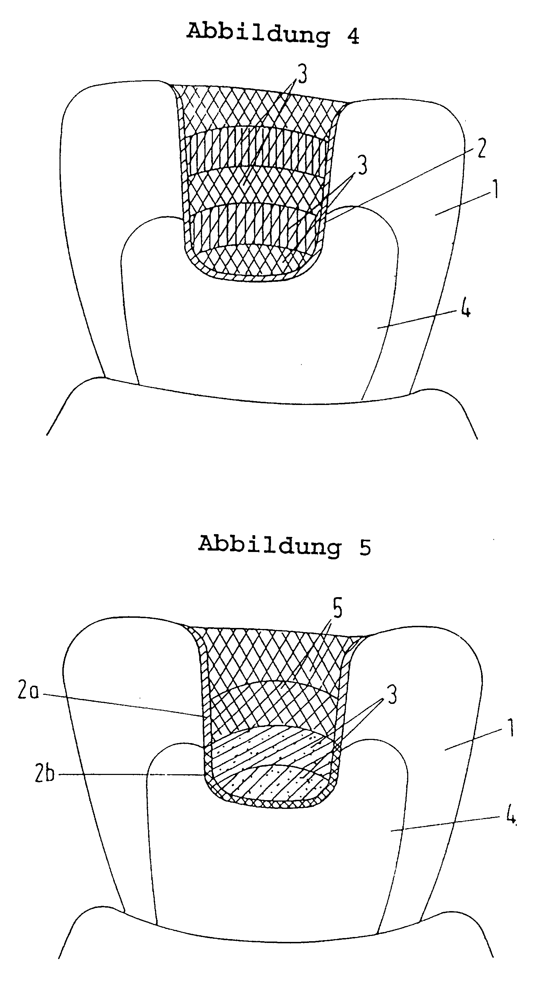

Dies ist in Abb. 4 dargestellt, worin (1) Zahnschmelz und (4) Dentin sind, (2) die Haftvermittler-Schicht und (3) die fließfähige Zusammensetzung (Compomer) in aufgebrachten Schichten darstellen.

Eine weitere Möglichkeit, dieses Verfahren zu verfeinern, besteht darin, die Kavität nur bis zum Dentinrand mit dem fließfähigen Compomer zu füllen und darauf eine Schicht eines stopfbaren Composites oder Compomers zu geben und wieder mit Licht zu härten. Als obere Schicht wird hier bevorzugt ein Composit eingesetzt, z.B. Ecusit® Composite von der Firma DMG. Bei dieser Methode gibt es auch die Möglichkeit, mit zwei verschieden Haftvermittlern für Dentin und Schmelz zu arbeiten.

Dies ist in Abb. 5 dargestellt, worin (1), (3) und (4) die gleiche Bedeutung wie in Abb. 4 haben, und (2a), (2b) zwei verschiedene Haftvermittler sowie (5) ein stopffähiges Composite darstellen.

Diese Technik ist für alle Kavitätenklassen anwendbar. Die Kombination von zwei Materialen unterschiedlicher Konsistenz bewährt sich vor allem in Klasse II Kavitäten, selbst wenn ein approximaler Anteil im Dentin liegt.

Bei den %-Angaben der Beschreibung handelt es sich stets um Gew.%-Angaben.

## Patentansprüche

1. Zusammensetzung zur Zahnrestaurierung bestehend aus einer Mischung in der Form einer dünnflüssigen Paste aus
(a) 25 bis 60 Gew.% polymerisierbarer Harzmischung enthaltend
- 3 bis 90 Gew.% einer oder mehrerer polymerisierbaren Säuren,
(b) 40 bis 75 Gew.% einer Füllstoffmischung, enthaltend
- 1 bis 5 Gew.% mikrofeines Siliciumdioxid,
- 10 bis 99 Gew.% ionenfreisetzenden Füllstoff und
- 0 bis 80 Gew.% inerten röntgenopaken Füllstoff,
und
(c) lichthärtendes Startersystem.

2. Zusammensetzung nach Anspruch 1, worin die Harzmischung 10 bis 50 Gew.% polymerisierbare Säuren enthält.

3. Zusammensetzung nach Anspruch 1 oder 2, worin die polymerisierbaren Säuren als Säuregruppen Carboxylgruppen, Phosphatreste, Phosponatreste, Schwefelsäurereste und/oder Sulfonatreste enthalten.

4. Zusammensetzung nach einem der Ansprüche 1 bis 3, worin das Molekulargewicht der polymerisierbaren Säuren kleiner als 1000 ist.

5. Zusammensetzung nach einem der vorhergehenden Ansprüche, worin die polymerisierbare Säure als polymerisierbare Komponente Acrylat- oder Methacrylatgruppen enthält.

6. Zusammensetzung nach einem der vorhergehenden Ansprüche, worin der ionenfreisetzende Füllstoff Fluorid freisetzt.

7. Zusammensetzung nach einem der vorhergehenden Ansprüche, worin der ionenfreisetzende Füllstoff ein Calcium-Aluminium-Fluor-Silikat ist.

8. Zusammensetzung nach einem der vorhergehenden Ansprüche, worin die Füllstoffmischung mindestens 50 Gew.% ionenfreisetzenden Füllstoff enthält.

9. Zusammensetzung nach einem der vorhergehenden Ansprüche, worin der ionenfreisetzende Füllstoff silanisiert ist.

10. Zusammensetzung nach einem der vorhergehenden Ansprüche, worin das mikrofeine Siliziumdioxid pyrogene Kieselsäure ist.

11. Zusammensetzung nach einem der vorhergehenden Ansprüche, worin der inerte Füllstoff silanisiert ist.

12. Verwendung einer Zusammensetzung zur Herstellung eines Medikamentes zur Füllung einer Zahnkavität, **dadurch gekennzeichnet, dass** die Zusammensetzung gemäß einem der vorhergehenden Ansprüche 1 bis 12 in die Zahnkavität aus einem Container durch eine schmale Kanüle direkt in die Kavität eingebracht wird und Lichthärten der Zusammensetzung im Zahn.

13. Verwendung nach Anspruch 12, wobei die Zusammensetzung aus einem Container, der aus einer Spritze mit aufgesetzter Kanüle besteht, eingebracht wird.

14. Verwendung nach Anspruch 12, wobei vorab die Kavität mit Haftvermittler vorbereitet wird.

15. Verwendung nach Anspruch 14, wobei vorab die Kavität im Dentin- und Schmelzbereich mit zwei unterschiedlichen Haftvermittlern vorbereitet wird.

16. Verwendung nach einem der Ansprüche 12 bis 15, **dadurch gekennzeichnet, dass** die dünnflüssige Mischung nur das Dentin ersetzt und darauf ein an sich bekannter polymerisierbarer Zement oder ein an sich bekanntes Composite einer festen Konsistenz geschichtet wird.

17. Container enthaltend die Zusammensetzung nach einem der Ansprüche 1 bis 11, wobei der Container eine schmale Kanüle mit Austrittsöffnung aufweist und vorzugsweise eine Spritze mit aufgesetzter Kanüle ist.

18. Container gemäß Anspruch 17, wobei der Container aus einem Einmalbehältnis mit schmaler, langer Austrittsöffnung besteht.

19. Container enthaltend die Zusammensetzung nach einem der Ansprüche 1 bis 11, wobei der Container ein Applikationsgerät ist, das eine Austrittsöffnung von kleiner 1,3 mm, vorzugsweise in Form einer Kanüle, aufweist, um die Zusammensetzung in die Kavität einzubringen.

## Claims

1. A composition for tooth restoration consisting of a mixture in the form of a liquid paste comprised of
(a) 25 to 60 %w/w of a polymerizable resin mixture comprising
- 3 to 90 %w/w of one or more polymerizable acids,
(b) 40 to 75 %w/w of a filler mixture, comprising
- 1 to 5 %w/w microfine silicon dioxide,
- 10 to 99 %w/w ion-releasing filler and
- 0 to 80 %w/w inert X-ray opaque filler
and
(c) light-hardening starter system.

2. The composition according to claim 1, wherein the resin mixture contains 10 to 50 %w/w polymerizable acids.

3. The composition according to claim 1 or 2, wherein the polymerizable acids contain carboxyl groups, phosphate residues, phosphonate residues, sulfuric acid residues and/or sulfonate residues as the acid groups.

4. The composition according to one of claims 1 to 3, wherein the molecular weight of the polymerizable acids is less than 1000.

5. The composition according to one of the preceding claims, wherein the polymerizable acid contains acrylate or methacrylate groups as the polymerizable component.

6. The composition according to one of the preceding claims, wherein the ion-releasing filler releases fluoride.

7. The composition according to one of the preceding claims, wherein the ion-releasing filler is a calcium-aluminum-fluorine-silicate.

8. The composition according to one of the preceding claims, wherein the filler mixture contains at least 50 %w/w of ion-releasing filler.

9. The composition according to one of the preceding claims, wherein the ion-releasing filler is silanized.

10. The composition according to one of the preceding claims, wherein the microfine silicon dioxide is pyrogenic silicic acid.

11. The composition according to one of the preceding claims, wherein the inert filler is silanized.

12. A use of a composition to manifacture a medicament to fill a dental cavity, **characterized in that** the composition according to one of the preceding claims 1 to 12 is introduced into the dental cavity from a container through a narrow cannula directly into the cavity, and the composition is light-hardened in the tooth.

13. The use according to claim 12, wherein the composition is introduced from a container consisting of a syringe with attached cannula.

14. The use according to claim 12, wherein the cavity is first prepared with an adhesion promoter.

15. The use according to claim 14, wherein the cavity is first prepared in the dentin and enamel area with two different adhesion promoters.

16. The use according to one of claims 12 to 15, **characterized in that** the liquid mixture replaces only the dentin, whereupon a known polymerizable cement or known composite having a solid consistency is layered over.

17. A container accommodating the composition according to one of claims 1 to 11, wherein the container exhibits a narrow cannula with outlet hole, and is preferably a syringe with attached cannula.

18. The container according to claim 17, wherein the container consists of a disposable container with a narrow, long outlet hole.

19. The container accommodating the composition according to one of claims 1 to 11, wherein the container is an applicator, which has an outlet hole smaller than 1.3 mm, preferably in the form of a cannula, to introduce the composition into the cavity.

## Revendications

1. Composition pour la restauration dentaire, constituée d'un mélange sous forme d'une pâte liquide constituée
(a) de 25 à 60% en poids d'un mélange polymérisable de résines contenant
- 3 à 90% en poids d'un ou de plusieurs acides polymérisables,
(b) de 40 à 75% en poids d'un mélange de charges contenant
- 1 à 5% en poids d'un dioxyde de silicium micronisé,
- 10 à 99% en poids d'une charge libérant des ions et
- 0 à 80% en poids d'une charge inerte opaque aux rayons X et
(c) d'un système d'initiation durcissant à la lumière.

2. Composition selon la revendication 1, dans laquelle le mélange de résines contient 10 à 50% en poids d'acides polymérisables.

3. Composition selon les revendications 1 ou 2, dans laquelle les acides polymérisables contiennent, comme groupements acides, des groupements carboxyle, des radicaux phosphate, des radicaux phosphonate, des radicaux acide sulfurique et/ou des radicaux sulfonate.

4. Composition selon l'une quelconque des revendications 1 à 3, dans laquelle le poids moléculaire des acides polymérisables est inférieur à 1000.

5. Composition selon l'une quelconque des revendications précédentes, dans laquelle l'acide polymérisable contient comme composant polymérisable des groupements acrylate ou méthacrylate.

6. Composition selon l'une quelconque des revendications précédentes, dans laquelle la charge libérant des ions libère du fluorure.

7. Composition selon l'une quelconque des revendications précédentes, dans laquelle la charge libérant des ions est un silicate de calcium, d'aluminium et de fluor.

8. Composition selon l'une quelconque des revendications précédentes, dans laquelle le mélange de charges contient au moins 50% en poids de charge libérant des ions.

9. Composition selon l'une quelconque des revendications précédentes dans laquelle la charge libérant des ions est silanisée.

10. Composition selon l'une quelconque des revendications précédentes, dans laquelle le dioxyde de silicium micronisé est de la silice pyrogène.

11. Composition selon l'une quelconque des revendications précédentes, dans laquelle la charge inerte est silanisée.

12. Utilisation d'une composition pour la préparation d'un médicament pour le remplissage d'une cavité dentaire, **caractérisée en ce que** la composition selon l'une quelconque des revendications précédentes 1 à 12 est introduite directement dans la cavité dentaire à partir d'un conteneur via une canule étroite et par le durcissement à la lumière de la composition dans la dent.

13. Utilisation selon la revendication 12, la composition étant introduite à partir d'un conteneur qui est constitué d'une seringue sur laquelle est placée une canule.

14. Utilisation selon la revendication 12, la cavité étant préparée au préalable avec un promoteur d'adhérence.

15. Utilisation selon la revendication 14, la cavité étant préparée au préalable dans la zone de la dentine et de l'émail avec deux promoteurs d'adhérence différents.

16. Utilisation selon l'une quelconque des revendications 12 à 15, **caractérisée en ce que** le mélange liquide ne remplace que la dentine et qu'on le recouvre par un ciment polymérisable connu en soi ou un matériau composite connu en soi d'une consistance solide.

17. Conteneur contenant la composition selon l'une quelconque des revendications 1 à 11, le conteneur présentant une canule étroite avec une ouverture de sortie et étant de préférence une seringue sur laquelle est placée une canule.

18. Conteneur selon la revendication 17, le conteneur étant constitué d'un récipient à usage unique présentant une ouverture de sortie étroite et allongée.

19. Conteneur contenant la composition selon l'une quelconque des revendications 1 à 11, le conteneur étant un appareil d'application qui présente une ouverture de sortie inférieure à 1,3 mm, de préférence sous forme d'une canule, pour introduire la composition dans la cavité.
